# EUROPEAN PATENT APPLICATION

(11) **EP 4 830 778 A1**
(43) Date of publication of application: **28.10.2026**
(21) Application number: 26173628.4
(22) Date of filing: 21.04.2026
(51) Int. Cl.: A61N 5/10

(54) **METHOD AND APPARATUS FOR PROVIDING A RADIATION TREATMENT PLAN**

(30) Priority: 22.04.2025 US 202519186186
(71) Applicant: Siemens Healthineers International AG, 6312 Steinhausen (CH)
(72) Inventor: SABEL, Martin, 5405 Dättwil (CH); RUOKOKOSKI, Emmi, 00100 Helsinki (FI); KUUSELA, Esa, 00100 Helsinki (FI); DONNELLY, Kellee, 00100 Helsinki (FI)
(74) Representative: Mathisen & Macara LLP

(57) **Abstract**

A control circuit accesses information regarding a time-based physiological limit for the particular patient (201) and uses that time-based physiological limit as an optimization objective 9202). The control circuit can then automatically split a treatment trajectory for the radiation treatment plan as a function of that time-based physiological limit (204).

## Description

### TECHNICAL FIELD

These teachings relate generally to treating a patient's target volume with energy pursuant to an energy-based treatment plan and more particularly to optimizing an energy-based treatment plan.

### BACKGROUND

The use of energy to treat medical conditions comprises a known area of prior art endeavor. For example, radiation therapy comprises an important component of many treatment plans for reducing or eliminating unwanted tumors. Unfortunately, applied energy does not inherently discriminate between unwanted material and adjacent tissues, organs, or the like that are desired or even critical to continued survival of the patient. As a result, energy such as radiation is ordinarily applied in a carefully administered manner to at least attempt to restrict the energy to a given target volume. A so-called radiation treatment plan often serves in the foregoing regards.

### SUMMARY OF THE INVENTION

A radiation treatment plan typically comprises specified values for each of a variety of treatment-platform parameters during each of a plurality of sequential fields. Treatment plans for radiation treatment sessions are often automatically generated through a so-called optimization process. As used herein, "optimization" will be understood to refer to improving a candidate treatment plan without necessarily ensuring that the optimized result is, in fact, the singular best solution. Such optimization often includes automatically adjusting one or more physical treatment parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects. The present invention provides a method for providing a radiation treatment plan for a particular patient, otherwise known as a radiation treatment plan optimization method, and radiation treatment apparatus, as defined in the claims.

Generally speaking, these various embodiments can provide a radiation treatment plan for a particular patient using a radiation treatment apparatus, wherein a control circuit accesses information regarding a time-based physiological limit for the particular patient and uses that time-based physiological limit as an optimization objective. The control circuit can then automatically split a treatment trajectory for the radiation treatment plan as a function of that time-based physiological limit. The present invention suitably provides an optimized radiation treatment plan for a particular patient. The present invention suitably provides a breast and/or lung radiation treatment plan optimization method.

The aforementioned time-based physiological limit can comprise a length of time that the particular patient can maintain stillness of a particular part of their body. That latter may comprise, for example, a length of time that the particular patient can hold their breath and thereby hold their upper torso still in those regards.

By one approach, the aforementioned treatment trajectory can include movement of a gantry along a predetermined path and/or movement of a patient support apparatus. These teachings will accommodate automatically splitting the treatment trajectory during any one or more of (i) prior to optimizing the radiation treatment plan, (ii) while optimizing the radiation treatment plan, and/or (iii) subsequent to optimizing the radiation treatment plan. By one approach, automatically splitting the treatment trajectory for the radiation treatment plan as a function of the time-based physiological limit can include identifying at least one preferred location at which to split the treatment trajectory.

By one approach, automatically splitting the treatment trajectory for the radiation treatment plan as a function of the time-based physiological limit can comprise splitting the treatment trajectory into a plurality of shorter treatment trajectory subsections to ensure that a planned radiation dose can be fully administered during each new planned field that corresponds to one of the shorter treatment trajectory subsections within a timeframe that corresponds to the time-based physiological limit. If desired, these teachings will accommodate automatically splitting the treatment trajectory for the radiation treatment plan as a function of the time-based physiological limit to include optimizing the radiation treatment plan to have a minimized number of treatment trajectory subsections.

By one approach, these teachings will further accommodate determining a dose delivery time that corresponds to the treatment trajectory. In such a case, these teachings can then further provide for determining to automatically split the treatment trajectory for the radiation treatment plan when the dose delivery time exceeds a threshold value that corresponds to the time-based physiological limit.

By one approach, these teachings can comprise a non-transitory computer-readable medium having a computer program that itself comprises instructions that, when the computer program is executed by a computer, causes the computer to carry out accessing information regarding a time-based physiological limit for the particular patient and using that time-based physiological limit as an optimization objective, followed by then automatically splitting a treatment trajectory for the radiation treatment plan as a function of that time-based physiological limit.

So configured, these teachings will then accommodate administering therapeutic radiation, to the particular patient, using a radiation treatment plan having an automatically split treatment trajectory.

So configured, these teachings can permit, for example, being able to automatically split treatment fields using a best imaging position aware method to thereby reduce total delivery time as well as unnecessary patient discomfort. Being able to automatically split the arc fields at low dose regions can also improve patient outcomes with more accurate dose delivery.

The radiation treatment apparatus may comprise a control circuit operably coupled to a memory, which memory may be integral to the control circuit or physically discrete from the control circuit. The memory may store information including, but not limited to, information for a particular patient, information regarding a particular radiation treatment platform, instructions for operation of the radiation treatment platform, one of more radiation treatment plans for a particular patient. Suitably, the control circuit is operably coupled to a user interface. The control circuit may be operably coupled to a network interface. Optionally, the control circuit is coupled to an imaging apparatus, which may be a computed tomography (CT) apparatus. The control circuit is preferably configured to output an optimized energy-based treatment plan, for a particular patient. The energy-based treatment plan typically comprises specified values for each of a variety of treatment-platform parameters during each of a plurality of sequential exposure fields. Suitably, the energy-based treatment plan is generated through an optimization process according to the present invention. The control circuit is suitably configured to control operation of the radiation treatment apparatus, including but not limited to, controlling movement and/or operation of one or more of a gantry, a radiation source, the patient support apparatus, a beam shaping apparatus.

In breast and lung radiation therapy treatments, breath-holding techniques are often used to limit the motion of the patient's organs during the treatment. In such treatments, the treatment planner usually needs to consider the patient's capability to reliably hold their breath for a useful period of time and plan the treatment in such a way that the treatment can be delivered in that patient's breath hold time in an accurate way. If the patient cannot hold their breath for the necessary amount of time, and instead starts breathing during delivery of the treatment, the treatment delivery will often be interrupted by a respiratory gating system. If this happens during a high dose rate part of the delivery trajectory, the correspondence of the delivered dose to the planned dose may be compromised. Furthermore, the applicant has determined that such interruptions can increase the total delivery time and contribute to unwanted patient discomfort.

In modern radiation treatment planning, the radiation treatment plan is typically created using an optimization algorithm that tries to minimize one or more cost functions that correspond to one or more user defined optimization objectives. In many cases, these objectives are dose objectives aiming to maximize target dose coverage and homogeneity while minimizing the dose to organs at risk. If desired, the time the patient can hold their breath can also be used as an optimization objective. For example, and as noted above, such an objective can serve a need to see that the delivery of individual fields takes less time than the patient's breath-hold time.

For arc-type treatments, the delivery time can depend at least in part on the arc length and dose fractionation. In some cases, a given patient's delivery time limit may be impossible to meet because the time it takes to deliver an unmodulated arc may already be very close to, or even longer than, the patient's time limit. In such a case, even an optimizer trying to meet a specified delivery time limit may violate such a time restriction. To avoid beam interruptions by a respiratory gating system in such an application setting, the human planner can manually split the arc into sub-fields where each sub-arc is short enough that, in combination with optimized modulation to meet the optimization objectives, the delivery time of each sub-field is shorter than the breath-hold time of the patient. Such an approach typically relies on a trial-and-error approach that continues until each sub-arc is short enough to meet the breath-hold time.

The teachings provided herein can provide for automatically splitting arcs to meet a given patient-defined time limit. By one approach, these teachings can automatically split the trajectory arc(s) while also optimizing the radiation treatment plan to achieve a minimum number of such subsections. For the sake of an illustrative example, this description presumes that this time-based physiological limit can correspond to a length of time that the particular patient can hold their breath. It will be understood, however, that these teachings will readily accommodate other time-based physiological limits. As one example in these regards, the time-based physiological limit can correspond to a length of time that the particular patient can hold some part of their body still notwithstanding that the particular patient may be experiencing considerable pain as may be the case when preparing a radiation treatment plan to serve as a palliative treatment.

### BRIEF DESCRIPTION OF DRAWINGS

Various needs in the foregoing regards are at least partially met through provision of the method and apparatus for providing a radiation treatment plan described in the following detailed description, particularly when studied in conjunction with the drawings, wherein:
FIG. 1 comprises a block diagram as configured in accordance with various embodiments of these teachings;
FIG. 2 comprises a flow diagram as configured in accordance with various embodiments of these teachings; and
FIG. 3 comprises a timing diagram as configured in accordance with various embodiments of these teachings.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

### DETAILED DESCRIPTION

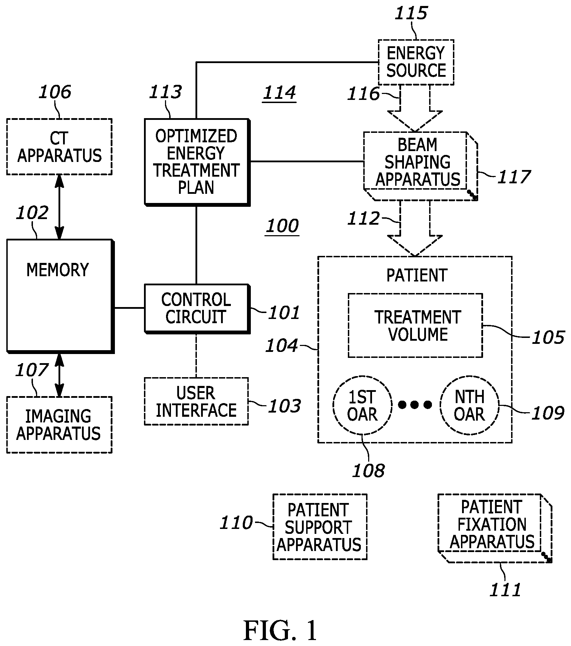

These and other benefits may become clearer upon making a thorough review and study of the following detailed description. Referring now to the drawings, and in particular to FIG. 1, an illustrative apparatus 100 that is compatible with many of these teachings will first be presented.

In this particular example, the enabling apparatus 100 includes a control circuit 101. Being a "circuit," the control circuit 101 therefore comprises structure that includes at least one (and typically many) electrically-conductive paths (such as paths comprised of a conductive metal such as copper or silver) that convey electricity in an ordered manner, which path(s) will also typically include corresponding electrical components (both passive (such as resistors and capacitors) and active (such as any of a variety of semiconductor-based devices) as appropriate) to permit the circuit to effect the control aspect of these teachings.

Such a control circuit 101 can comprise a fixed-purpose hard-wired hardware platform (including but not limited to an application-specific integrated circuit (ASIC) (which is an integrated circuit that is customized by design for a particular use, rather than intended for general-purpose use), a field-programmable gate array (FPGA), and the like) or can comprise a partially or wholly-programmable hardware platform (including but not limited to microcontrollers, microprocessors, and the like). These architectural options for such structures are well known and understood in the art and require no further description here. This control circuit 101 is configured (for example, by using corresponding programming as will be well understood by those skilled in the art) to carry out one or more of the steps, actions, and/or functions described herein.

It will be appreciated that the control circuit 101 may comprise a single integrated platform or may comprise a plurality of such circuits that work in cooperation with one another.

The control circuit 101 operably couples to a memory 102. This memory 102 may be integral to the control circuit 101 or can be physically discrete (in whole or in part) from the control circuit 101 as desired. This memory 102 can also be local with respect to the control circuit 101 (where, for example, both share a common circuit board, chassis, power supply, and/or housing) or can be partially or wholly remote with respect to the control circuit 101 (where, for example, the memory 102 is physically located in another facility, metropolitan area, or even country as compared to the control circuit 101). As with the control circuit 101, the memory 102 may comprise a singular structure or may comprise a plurality of memory platforms that collectively comprise the "memory" of this apparatus 100.

In addition to information such as optimization information for a particular patient and information regarding a particular radiation treatment platform as described herein, this memory 102 can serve, for example, to non-transitorily store the computer instructions that, when executed by the control circuit 101, cause the control circuit 101 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both nonvolatile memory (such as read-only memory (ROM) as well as volatile memory (such as a dynamic random access memory (DRAM)).

By one optional approach the control circuit 101 also operably couples to a user interface 103. This user interface 103 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

If desired the control circuit 101 can also operably couple to a network interface (not shown). So configured the control circuit 101 can communicate with other elements (both within the apparatus 100 and external thereto) via the network interface. Network interfaces, including both wireless and non-wireless platforms, are well understood in the art and require no particular elaboration here.

By one approach, a computed tomography apparatus 106 and/or other imaging apparatus 107 as are known in the art can source some or all of any desired patient-related imaging information.

In this illustrative example the control circuit 101 is configured to ultimately output an optimized energy-based treatment plan (such as, for example, an optimized radiation treatment plan 113). This energy-based treatment plan typically comprises specified values for each of a variety of treatment-platform parameters during each of a plurality of sequential exposure fields. In this case the energy-based treatment plan is generated through an optimization process, examples of which are provided further herein.

By one approach the control circuit 101 can operably couple to an energy-based treatment platform 114 that is configured to deliver therapeutic energy 112 to a corresponding patient 104 having at least one treatment volume 105 and also one or more organs-at-risk (represented in FIG. 1 by a first through an Nth organ-at-risk 108 and 109) in accordance with the optimized energy-based treatment plan 113. These teachings are generally applicable for use with any of a wide variety of energy-based treatment platforms/apparatuses. In a typical application setting the energy-based treatment platform 114 will include an energy source such as a radiation source 115 of ionizing radiation 116.

By one approach this radiation source 115 can be selectively moved via a gantry along an arcuate pathway (where the pathway encompasses, at least to some extent, the patient themselves during administration of the treatment). The arcuate pathway may comprise a complete or nearly complete circle as desired. By one approach the control circuit 101 controls the movement of the radiation source 115 along that arcuate pathway, and may accordingly control when the radiation source 115 starts moving, stops moving, accelerates, de-accelerates, and/or a velocity at which the radiation source 115 travels along the arcuate pathway.

As one illustrative example, the radiation source 115 can comprise, for example, a radio-frequency (RF) linear particle accelerator-based (linac-based) x-ray source. A linac is a type of particle accelerator that greatly increases the kinetic energy of charged subatomic particles or ions by subjecting the charged particles to a series of oscillating electric potentials along a linear beamline, which can be used to generate ionizing radiation (e.g., X-rays) 116 and high energy electrons.

A typical energy-based treatment platform 114 may also include one or more support apparatuses 110 (such as a couch) to support the patient 104 during the treatment session, one or more patient fixation apparatuses 111, a gantry or other movable mechanism to permit selective movement of the radiation source 115, and one or more energy-shaping apparatuses (for example, beam-shaping apparatuses 117 such as jaws, multi-leaf collimators, and so forth) to provide selective energy shaping and/or energy modulation as desired.

In a typical application setting, it is presumed herein that the patient support apparatus 110 is selectively controllable to move in any direction (i.e., any X, Y, or Z direction) during an energy-based treatment session by the control circuit 101. As the foregoing elements and systems are well understood in the art, further elaboration in these regards is not provided here except where otherwise relevant to the description.

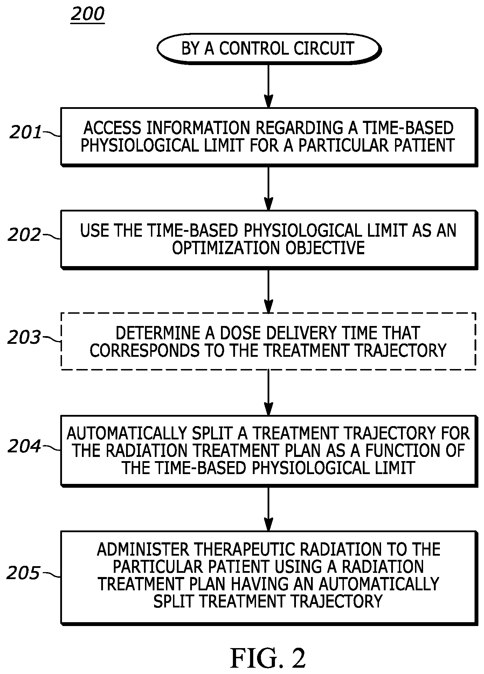

Referring now to FIG. 2, a process 200 that can be carried out, for example, in conjunction with the above-described application setting (and more particularly via the aforementioned control circuit 101 will be described. Generally speaking, this process 200 serves to facilitate providing an optimized radiation treatment plan 113 to thereby facilitate treating a particular patient with therapeutic radiation using a particular radiation treatment platform per that optimized radiation treatment plan.

This process 200 can serve to provide a radiation treatment plan for a particular patient using a radiation treatment apparatus such as described above. At block 201, the control circuit 101 can access information regarding a time-based physiological limit for the particular patient. The foregoing may comprise, for example, the control circuit 101 accessing the aforementioned memory 102 for that previously-stored information. That time-based physiological limit can comprise, for example, a length of time that the particular patient can maintain stillness of a particular part of their body. The time-based physiological limit may be expressed, for example, in seconds.

At block 202, the control circuit 101 uses the aforementioned time-based physiological limit as an optimization objective. An optimization objective refers to a predefined goal or target that the optimization process aims to achieve or approximate. Objectives are quantifiable criteria that guide the optimization algorithm in adjusting the parameters of the treatment plan to meet specific clinical requirements. These objectives can typically include maximizing the dose to the tumor while minimizing the exposure to surrounding healthy tissues and critical organs, achieving a homogeneous dose distribution within the target volume, or meeting dose-volume constraints that are established based on clinical guidelines and patient-specific factors. In this case, this additional objective can seek to maintain the achievement of other goals while also taking into account the time-based limitation of the time-based physiological limit. Objectives are typically translated into mathematical expressions that form part of the cost function or fitness function, which the optimization algorithm seeks to minimize or maximize during the iterative process. By evaluating the treatment plan against these objectives at each iteration, the algorithm can iteratively refine the plan parameters, such as beam angles, intensities, and shapes, to converge towards an optimal treatment plan that best satisfies a balance between treatment efficacy, patient safety, and the timeframe limitations of the time-based physiological limit.

There are a number of optimization approaches available in the art. As the present teachings are not overly sensitive to any particular choice in these regards, further elaboration regarding any particular optimization approach is not provided here for the sake of brevity except where otherwise provided herein.

If desired, and as illustrated at optional block 203, these teachings can accommodate the control circuit also determining a dose delivery time that corresponds to the treatment trajectory. That dose delivery time, when available, can be utilized as described below.

At block 204, the control circuit can then automatically split a treatment trajectory for the radiation treatment plan as a function of the aforementioned time-based physiological limit. That treatment trajectory may comprise, for example, movement of a gantry along a predetermined path and/or movement of a patient support apparatus such as a treatment platform couch that is capable of movement in one or more directions during the treatment process.

It should be noted that this automatic splitting of the treatment trajectory can occur prior to optimizing the radiation treatment plan, while optimizing the radiation treatment plan, and/or subsequent to optimizing the radiation treatment plan as desired.

By one approach, automatically splitting the treatment trajectory for the radiation treatment plan as a function of the time-based physiological limit can comprise splitting the treatment trajectory into a plurality of shorter treatment trajectory subsections to ensure that a planned radiation dose can be fully administered during each new planned field that corresponds to one of the shorter treatment trajectory subsections within a time frame that corresponds to the time-based physiological limit (for example, within a time frame having a time duration that is less than a time duration that is specified by the time-based physiological limit). The foregoing can include, if desired, identifying at least one preferred location at which to split the treatment trajectory into two or more subsections.

Automatically splitting the treatment trajectory as a function of the time-based physiological limit can include, if desired, optimizing the radiation treatment plan to have a minimized number of treatment trajectory subsections. So configured, the optimization process, while serving to accommodate splitting the treatment trajectory into subsections, will nevertheless also be biased towards minimizing the number of splits that are utilized.

When the control circuit 101 has determined a dose delivery time that corresponds to the treatment trajectory as described above in block 203, these teaching can then also accommodate determining to automatically split the treatment trajectory for the radiation treatment plan when the dose delivery time exceeds a threshold value that corresponds to the time-based physiological limit. As a simple example in these regards, when the time-based physiological limit represents that the particular patient can only reliably hold their breath for 15 seconds, a threshold value of 13 seconds may be utilized in the foregoing regards. In that case, when the dose delivery time is longer than the 13 seconds threshold value, the control circuit 101 can responsively determine to automatically split the treatment trajectory for the radiation treatment plan per these teachings.

At block 205, these teachings will accommodate, if desired, administering therapeutic radiation to the particular patient using a radiation treatment plan having an automatically split treatment trajectory per the foregoing teachings.

Further details that comport with these teachings will now be presented. It will be understood that the specific details of these examples are intended to serve an illustrative purpose and are not intended to suggest any particular limitations with respect to these teachings.

By one approach, the time required to deliver the full trajectory can be estimated prior to multi-leaf collimator leaf-position and monitor unit optimization. A lower bound for the time estimate can be calculated for an arbitrary subsection by splitting the subsection into small intervals, and at every interval then determining the trajectory axis (corresponding to, for example, gantry rotation, multi-leaf collimator rotation, and patient support surface degrees-of-freedom) that requires the longest time to travel the specified distance with an axis-specific maximum speed.

As an example in those regards, for a simple arc-field (where the gantry is the only moving axis) the minimum time required to deliver a sub-arc can be calculated by first determining the difference between the gantry positions in the beginning and end points of the sub-arc, and then dividing this by the gantry's maximum velocity.

More accurate estimates can also take into account the finite acceleration of the relevant axis in the beginning and end of the subsection (assuming that the subsection starts and ends so that the machine is at rest (and hence there is no moving axis those points). And an even more accurate estimation can be calculated by also taking into account how much time is required to be spent to deliver the required amount of dose to the target(s) when the maximum dose rate is known.

By one approach, when it turns out that the minimum time for the full trajectory is longer than the desired maximum delivery time of a single field, splitting of the trajectory can occur automatically. By one approach, the trajectory splitting logic can first determine the minimum number of subsections. By another approach, these teachings will also accommodate considering a small increase to the minimum number of subsections (such as by increasing that minimum number by 1) to allow more flexibility as regards the splitting position(s).

These teachings will also accommodate deducing optimal splitting positions. One approach in those regards comprises a best imaging position aware approach. Often, the anterior-posterior or posterior-anterior directions and the right-left or left-right directions are best for kV or MV imaging. Adding trajectory split points automatically at those directions can help avoid losing time at patient position checks and/or can use input from a kilovoltage fluoroscopy machine to trigger a next breath hold based on internal object tracking (of the patient's diaphragm, for example). (A kilovoltage fluoroscopy machine is an imaging device used in medical settings to provide real-time moving X-ray images of a patient's internal structures.) Additional splits might be needed to meet the breath-hold time restriction.

By another approach, the arc split positions can be defined automatically based on a complexity analysis of the corresponding fluence maps early in the optimization process in conjunction with the breath-hold time restriction. There are at least two factors that can be helpful to consider. First, how quickly the fluence varies in certain direction, and second, how much dose is delivered from the corresponding direction. By one approach, the location of the arc split can be set to regions where, at the same time, the fluence map varies slowly and not much dose is being delivered. Such an arc split location can allow a smoother stop of the radiation beam when reaching the split point and re-starting when the patient is in a subsequent breath-hold cycle.

And by yet another approach, these teachings can take into account an efficiency criterion. For example, the splitting logic might consider only such locations that will minimize the overall number of splits to thereby reduce the number of breath-hold cycles (and time) needed to deliver the full radiation treatment plan. In some cases, it might be useful to allow some overlap between split subsections (for example, where the end of a previous subsection overlaps with the beginning of a next subsection). The amount and/or permitted extent of such an overlap could be evaluated using an efficiency criterion (or criteria) of choice.

With trajectory subsections automatically created, the corresponding control points can now be optimized using, for each subsection, a corresponding breath-hold time limit. The foregoing may comprise, for example, restricting the motion of the optimized degrees-of-freedoms (such as multi-leaf collimator leaf positions and/or delivered monitor units) so that such movement does not unduly slow down the trajectory, and/or by using explicit time constraint penalizing solutions whose estimated time is larger than (or close to) the defined maximum delivery time.

If desired, defining split locations can happen later in the optimization process when, for example, the overall delivery time estimate, and the locations of the low and high dose regions are more accurate. The same information as mentioned above can be derived from the already optimized control points and dose, and a second optimization round can then be triggered for the split arcs. At least one benefit of this approach is that the estimates for the delivery time will typically be more accurate and hence no large thresholds may be needed to meet the delivery time. This approach can also serve to reduce the overall number of splits. And, in addition, the probability of meeting the corresponding breath-hold time limit for all sub-arcs is higher than when splitting the arcs earlier in the optimization process.

By one approach, to reduce the amount of fluence map or delivery complexity analysis, these teachings will accommodate reducing the search space to regions one might denote as maximum breath-hold time - X seconds (where, for example, if X is ¼ of the maximum breath-hold time, the region is still large enough to find a split point.) A next split point can then be specified as that just-determined split point + maximum breath-hold time - X seconds. This approach may help as well to keep the overall number of split points relatively small.

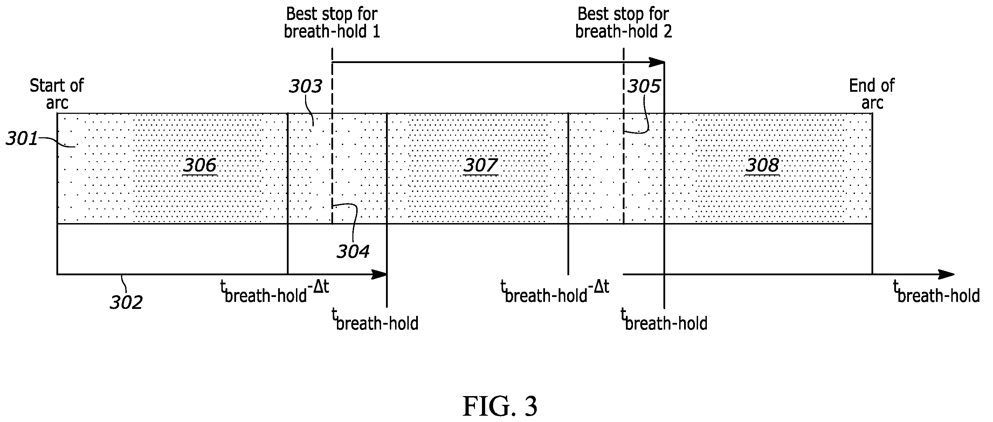

FIG. 3 presents an illustrative arc 301 having split points. In this example, to determine each split point a maximum length of the sub-arc is shown by the t_{breath-hold} time arrow 302. The region 303 where the split can be defined is determined by t_{breath-hold} - Δt. A best stop point 304 for the arc 301 can then be determined in this region 303. In cases where the remaining arc length takes longer than t_{breath-hold}, additional stop points 305 can be defined in the same way. Using this approach, FIG. 3 presents an arc 301 having two stop points 304, 305 and three resultant sub arcs 306, 307, and 308.

Further aspects of these teachings are provided by the subject matter of the following clauses (where it will be understood that any of these clauses can be combined with any one or more of the other clauses as desired).

Clause 1. A method for providing a radiation treatment plan for a particular patient using a radiation treatment apparatus, the method comprising: by a control circuit: accessing information regarding a time-based physiological limit for the particular patient; using the time-based physiological limit as an optimization objective; automatically splitting a treatment trajectory for the radiation treatment plan as a function of the time-based physiological limit.

Clause 2. The method of clause 1 wherein the time-based physiological limit comprises a length of time that the particular patient can maintain stillness of a particular part of their body.

Clause 3. The method of either of clause 1 or 2 wherein the treatment trajectory includes movement of a gantry along a predetermined path.

Clause 4. The method of any of clause 1 through 3 wherein the treatment trajectory includes movement of a patient support apparatus.

Clause 5. The method of any of clause 1 through 4 wherein automatically splitting the treatment trajectory occurs during one of: prior to optimizing the radiation treatment plan; while optimizing the radiation treatment plan; subsequent to optimizing the radiation treatment plan.

Clause 6. The method of any of clause 1 through 5 wherein automatically splitting the treatment trajectory for the radiation treatment plan as a function of the time-based physiological limit comprises splitting the treatment trajectory into a plurality of shorter treatment trajectory subsections to ensure that a planned radiation dose can be fully administered during each new planned field that corresponds to one of the shorter treatment trajectory subsections within a timeframe that corresponds to the time-based physiological limit.

Clause 7. The method of any of clause 1 through 6 wherein automatically splitting the treatment trajectory for the radiation treatment plan as a function of the time-based physiological limit includes optimizing the radiation treatment plan to have a minimized number of treatment trajectory subsections.

Clause 8. The method of any of clause 1 through 7 further comprising:
determining a dose delivery time that corresponds to the treatment trajectory;
determining to automatically split the treatment trajectory for the radiation treatment plan when the dose delivery time exceeds a threshold value that corresponds to the time-based physiological limit.

Clause 9. The method of any of clause 1 through 8 wherein automatically splitting the treatment trajectory for the radiation treatment plan as a function of the time-based physiological limit includes identifying at least one preferred location at which to split the treatment trajectory.

Clause 10. The method of any of clause 1 through 9 further comprising:
administering therapeutic radiation to the particular patient using a radiation treatment plan having an automatically split treatment trajectory.

Clause 11. An apparatus for providing a radiation treatment plan for a particular patient using a radiation treatment apparatus, the apparatus comprising: a control circuit configured to: access information regarding a time-based physiological limit for the particular patient; use the time-based physiological limit as an optimization objective; automatically split a treatment trajectory for the radiation treatment plan as a function of the time-based physiological limit.

Clause 12. The apparatus of clause 11 wherein the time-based physiological limit comprises a length of time that the particular patient can maintain stillness of a particular part of their body.

Clause 13. The apparatus of either of clause 11 or 12 wherein the treatment trajectory includes movement of a gantry along a predetermined path.

Clause 14. The apparatus of any of clause 11 through 13 wherein the treatment trajectory includes movement of a patient support apparatus.

Clause 15. The apparatus of any of clause 11 through 14 wherein the control circuit is configured to automatically split the treatment trajectory occurs during one of: prior to optimizing the radiation treatment plan; while optimizing the radiation treatment plan; subsequent to optimizing the radiation treatment plan.

Clause 16. The apparatus of any of clause 11 through 15 wherein the control circuit is configured to automatically split the treatment trajectory for the radiation treatment plan as a function of the time-based physiological limit by splitting the treatment trajectory into a plurality of shorter treatment trajectory subsections to ensure that a planned radiation dose can be fully administered during each new planned field that corresponds to one of the shorter treatment trajectory subsections within a timeframe that corresponds to the time-based physiological limit.

Clause 17. The apparatus of any of clause 11 through 16 wherein the control circuit is configured to automatically split the treatment trajectory for the radiation treatment plan as a function of the time-based physiological limit by optimizing the radiation treatment plan to have a minimized number of treatment trajectory subsections.

Clause 18. The apparatus of any of clause 11 through 17 wherein the control circuit is further configured to: determine a dose delivery time that corresponds to the treatment trajectory; determine to automatically split the treatment trajectory for the radiation treatment plan when the dose delivery time exceeds a threshold value that corresponds to the time-based physiological limit.

Clause 19. The apparatus of any of clause 11 through 18 wherein the control circuit is configured to automatically split the treatment trajectory for the radiation treatment plan as a function of the time-based physiological limit by identifying at least one preferred location at which to split the treatment trajectory.

Clause 20. The apparatus of any of clause 11 through 19 wherein the control circuit is further configured to: administer therapeutic radiation to the particular patient using a radiation treatment plan having an automatically split treatment trajectory.

Those skilled in the art will recognize that a wide variety of modifications, alterations, and combinations can be made with respect to the above-described embodiments without departing from the scope of the invention, and that such modifications, alterations, and combinations are to be viewed as being within the ambit of the inventive concept.

## Claims

1. A radiation treatment plan optimization method for a particular patient (200), the method comprising:
by a control circuit (101):
accessing information regarding a time-based physiological limit for the particular patient (201);
using the time-based physiological limit as an optimization objective (202);
automatically splitting a treatment trajectory for the radiation treatment plan as a function of the time-based physiological limit (204).

2. The method of claim 1 wherein the time-based physiological limit comprises a length of time that the particular patient can maintain stillness of a particular part of their body.

3. The method of claim 1 or 2, wherein the treatment trajectory includes movement of a gantry along a predetermined path and/or wherein the treatment trajectory includes movement of a patient support apparatus.

4. The method of claim 1, 2 or 3, wherein automatically splitting the treatment trajectory occurs during one of:
(i) prior to optimizing the radiation treatment plan;
(ii) while optimizing the radiation treatment plan;
(iii) subsequent to optimizing the radiation treatment plan.

5. The method of any one of the preceding claims, wherein automatically splitting the treatment trajectory for the radiation treatment plan as a function of the time-based physiological limit (204) comprises splitting the treatment trajectory into a plurality of shorter treatment trajectory subsections to ensure that a planned radiation dose can be fully administered during each new planned field that corresponds to one of the shorter treatment trajectory subsections within a timeframe that corresponds to the time-based physiological limit, and optionally, automatically splitting the treatment trajectory for the radiation treatment plan as a function of the time-based physiological limit (204) includes optimizing the radiation treatment plan to have a minimized number of treatment trajectory subsections.

6. The method of any one of the preceding claims further comprising:
determining a dose delivery time that corresponds to the treatment trajectory (203);
determining to automatically split the treatment trajectory for the radiation treatment plan when the dose delivery time exceeds a threshold value that corresponds to the time-based physiological limit.

7. The method of any one of the preceding claims, wherein automatically splitting the treatment trajectory for the radiation treatment plan as a function of the time-based physiological limit (204) includes identifying at least one preferred location at which to split the treatment trajectory.

8. A radiation treatment apparatus (100) for providing a radiation treatment plan for a particular patient, comprising a radiation treatment apparatus, the apparatus comprising:
a control circuit (110) configured to:
access information regarding a time-based physiological limit for the particular patient (201);
use the time-based physiological limit as an optimization objective (202);
automatically split a treatment trajectory for the radiation treatment plan as a function of the time-based physiological limit (204).

9. The apparatus of claim 8 wherein the time-based physiological limit comprises a length of time that the particular patient can maintain stillness of a particular part of their body.

10. The apparatus of claim 8 or 9 wherein the treatment trajectory includes movement of a gantry along a predetermined pathand/or the treatment trajectory includes movement of a patient support apparatus.

11. The apparatus of claim 8, 9 or 10 wherein the control circuit is configured to automatically split the treatment trajectory occurs during one of:
(i) prior to optimizing the radiation treatment plan;
(ii) while optimizing the radiation treatment plan;
(iii) subsequent to optimizing the radiation treatment plan.

12. The apparatus of any one of claims 8 to 11 wherein the control circuit is configured to automatically split the treatment trajectory for the radiation treatment plan as a function of the time-based physiological limit (204) by splitting the treatment trajectory into a plurality of shorter treatment trajectory subsections to ensure that a planned radiation dose can be fully administered during each new planned field that corresponds to one of the shorter treatment trajectory subsections within a timeframe that corresponds to the time-based physiological limit.

13. The apparatus of any one of claims 8 to 12 wherein the control circuit is configured to automatically split the treatment trajectory for the radiation treatment plan as a function of the time-based physiological limit (204) by optimizing the radiation treatment plan to have a minimized number of treatment trajectory subsections.

14. The apparatus of any one of claims 8 to 13 wherein the control circuit is further configured to:
determine a dose delivery time that corresponds to the treatment trajectory (203);
determine to automatically split the treatment trajectory for the radiation treatment plan when the dose delivery time exceeds a threshold value that corresponds to the time-based physiological limit (204).

15. The apparatus of any one of claims 8 to 14 wherein the control circuit is configured to automatically split the treatment trajectory for the radiation treatment plan as a function of the time-based physiological limit (204) by identifying at least one preferred location at which to split the treatment trajectory.
